# EUROPEAN PATENT APPLICATION

(11) **EP 2 818 500 A2**
(43) Date of publication of application: **31.12.2014**
(21) Application number: 14165643.9
(22) Date of filing: 23.04.2014
(51) Int. Cl.: C08J 9/28

(54) **Polysaccharide Monolithic Structure and Manufacturing Method Therefor**

(30) Priority: 23.04.2013 JP 2013090229; 28.10.2013 JP 2013223567
(71) Applicant: JNC Corporation, Chiyoda-ku Tokyo 100-8105 (JP)
(72) Inventor: Nakama, Tsuyoshi, Kanagawa, 236-8605 (JP); Todokoro, Masami, Kanagawa, 236-8605 (JP); Iwamoto, Eri, Kanagawa, 236-8605 (JP)
(74) Representative: Nicholls, Kathryn Margaret

(57) **Abstract**

A problem is to provide a monolithic structure that is a porous body formed of a polysaccharide being a naturally-occurring polymer, and has continuous pores having an average pore diameter suitable for biomolecule separation to allow formation into an arbitrary shape, and a manufacturing method therefor. A solution is to manufacture the polysaccharide monolithic structure by a method including a first step for dissolving a polysaccharide into a mixed solvent including a solvent into which the polysaccharide is soluble and a solvent into which the polysaccharide is insoluble, at a temperature lower than a boiling point of the mixed solvent, to give a polysaccharide solution, and a second step for cooling the polysaccharide solution to give the polysaccharide monolithic structure. The polysaccharide monolithic structure obtained is a porous body having continuous pores having an average pore diameter of 0.01 to 20 micrometers and a thickness of 100 micrometers or more.

## Description

The invention relates to a polysaccharide monolithic structure and a manufacturing method therefor. The invention further relates to a purification method for an arbitrary objective substance using the polysaccharide monolithic structure.

Attention is focused on research and development of a biomedicine manufactured by using various animals and insects, and cells thereof and microorganisms as a host. Productivity of the biomedicine is improving by high-capacity culture and high-titer culture, and achievement of high efficiency is required also for a purification step as associated therewith. The biomedicine is of biological origin, and therefore a very sophisticated purification technology is needed, and purification is made in combination of centrifugal separation, ultrafiltration, microfiltration, chromatography and so forth. In particular, the chromatography is a core technology of purification.

As a packing material for chromatography used for separating protein, nucleic acid, a virus or the like to be used as a biomedicine raw material, porous particles having various pore diameters or porous particles into which a functional group is introduced in order to impart adsorption performance are manufactured and commercially available. A naturally-occurring polymer such as cellulose is used for most of the packing materials for chromatography.

However, most of commercial items are available mainly for the purpose of separating protein having a molecular weight of approximately several hundreds of thousands, and only a limited item is suitable for a giant biopolymer such as nucleic acid having a molecular weight of one million or more, which is caused by difficulty in increasing a pore diameter without adversely affecting pressure-resistant strength of particles.

Incidentally, as a porous material, attention is drawn to a monolith being a single block of porous body continuously having three-dimensional network structure skeletons and voids, respectively, as a next-generation porous material. In the monolith, a diameter of the skeletons and a diameter of the pores to serve as channels can be independently controlled.

For example, a silica monolith formed using silica as a material is commercially available as a monolith column, and has high separability and excellent mechanical strength. Moreover, technology development is progressing for controlling a skeleton diameter and a channel pore and controlling surface micropores.

However, most of the technology development is directed to a capillary-sized silica monolith column having an inner diameter of 0.3 mm or less due to ease of preparation of a capillary-sized monolith column. More specifically, such a silica monolith is intended for analysis and does not have adsorption capacity that can be used for the purification step in biomedicine production.

In addition to silica, various reports have been made so far on a monolithic structure formed of a polymer material and a manufacturing method therefor. Patent literature No. 1 and Patent literature No. 2 disclose a monolithic structure prepared using a synthetic polymer material as a raw material, and Patent literature No. 3 discloses a monolithic structure prepared using biodegradable polylactic acid as a main component.

However, only a limited report has been made on a monolithic structure formed of a polysaccharide being a naturally-occurring polymer. More specifically, no report has been made on a monolithic structure having a polysaccharide skeleton that has a large number of hydroxyl groups and is expected to have low interaction with a biological substance such as protein.

A porous membrane made from a cellulose derivative being a polysaccharide derivative is used in various fields such as purification of water including drinking water and industrial water, substance separation and purification in each step of chemical industry, pharmaceutical industry and food industry, and in a medical field including hemodialysis by an artificial kidney.

Patent literature No. 4 discloses a heat-induced phase separation method using a high boiling point solvent as a manufacturing method for a porous membrane using a cellulose derivative. However, a porous membrane obtained by the method disclosed in Patent literature No. 4 is porous, but the pores include no continuous pores but discontinuous pores, individually. Therefore, the membrane is hard to be utilized as a column material. Moreover, the membrane is in the form of a 1 to 100 micrometer-thick membrane to allow no manufacture of a formed body having an arbitrary shape.

### Citation List

### Patent Literature

Patent literature No. 1: JP 2009-030017 A.
Patent literature No. 2: JP 2012-107216 A.
Patent literature No. 3: JP 2010-260952 A.
Patent literature No. 4: JP 2003-001074 A.

In view of such a situation, embodiments of the invention desirably provide a monolithic structure that is a porous body formed of a polysaccharide being a naturally-occurring polymer, and has continuous pores having an average pore diameter suitable for biomolecule separation to allow formation into an arbitrary shape, and a manufacturing method therefor.

The present inventors have found that a monolithic structure can be easily manufactured from a polysaccharide by selecting a suitable solvent, and thus have completed the invention based on the finding.

More specifically, aspects of the invention include those described below.
Item 1. A polysaccharide-containing monolithic structure, wherein the polysaccharide monolithic structure is a porous body having continuous pores having an average pore diameter of 0.01 to 20 micrometers, and a thickness of 100 micrometers or more.
Item 2. The polysaccharide monolithic structure according to item 1, wherein the polysaccharide is cellulose.
Item 3. The polysaccharide monolithic structure according to item 1 or 2, wherein at least one hydroxyl group of the polysaccharide is esterified.
Item 4. The polysaccharide monolithic structure according to any one of items 1 to 3, including cross-linked structure.
Item 5. The polysaccharide monolithic structure according to any one of items 1 to 4, wherein a ligand is introduced into at least one hydroxyl group of the polysaccharide.
Item 6. A manufacturing method for a polysaccharide monolithic structure, comprising:
   a first step for dissolving a polysaccharide into a mixed solvent including a solvent into which the polysaccharide is soluble and a solvent into which the polysaccharide is insoluble, at a temperature lower than a boiling point of the mixed solvent, to give a polysaccharide solution; and
   a second step for cooling the polysaccharide solution to give the polysaccharide monolithic structure.
Item 7. The method according to item 6, wherein a third component is added to the mixed solvent.
Item 8. The manufacturing method according to item 7, wherein a concentration of the third component in the mixed solvent is 0.1 to 5% by weight.
Item 9. The manufacturing method according to item 7 or 8, wherein the third component is polyethylene glycol.
Item 10. The manufacturing method according to any one of items 6 to 9, wherein a volume ratio of the solvent into which the polysaccharide is soluble and the solvent into which the polysaccharide is insoluble in the mixed solvent is 5 : 95 to 60 : 40.
Item 11. The manufacturing method according to any one of items 6 to 10, wherein a concentration of the polysaccharide in the mixed solvent is 0.1 to 30% by weight.
Item 12. The manufacturing method according to any one of items 6 to 11, wherein, in the second step, the polysaccharide solution is cooled to a temperature lower by 5 to 200°C than a temperature before cooling.
Item 13. The manufacturing method according to any one of items 6 to 12, wherein the polysaccharide is esterified cellulose.
Item 1.4. A manufacturing method for a saponified polysaccharide monolithic structure, comprising a third step, after a polysaccharide monolithic structure is manufactured by the manufacturing method according to any one of items 6 to 13, to be applied for saponifying the polysaccharide monolithic structure after the second step.
Item 15. A manufacturing method for a cross-linked and/or ligand-introduced polysaccharide monolithic structure, comprising a fourth step, after a saponified polysaccharide monolithic structure is manufactured by the manufacturing method according to item 14, to be applied for applying cross-linking and/or ligand-introducing treatment after the third step.
Item 16. A purification method for an objective substance, using the polysaccharide monolithic structure according to any one of items 1 to 5.

The invention can provide a monolithic structure having a pore diameter suitable for biopolymer separation from an inexpensive polysaccharide or polysaccharide derivative. Moreover, the invention can arbitrarily provide the monolithic structure with adsorption performance to the biopolymer by undergoing a step of saponification, cross-linking and ligand introduction, and further can provide a material useful for a purification method for an objective substance using the monolithic structure.

Embodiments of the invention are described herein by way of example, and illustrated in the accompanying figures, of which:
FIG. 1 is a SEM photograph of a cross section of a monolithic structure in Manufacture example 1.
FIG. 2 is a SEM photograph of a cross section of a monolithic structure in Manufacture Example 4.
FIG. 3 is a SEM photograph of a cross section of a monolithic structure in Manufacture Example 5.
FIG. 4 is a SEM photograph of a cross section of a monolithic structure in Manufacture Example 8.
FIG. 5 is a SEM photograph of a cross section of a monolithic structure in Manufacture Example 17.
FIG. 6 is a graph showing a relationship between a flow rate and pressure during liquid permeation in Test Example 2.

Embodiments of the invention are now described.

### (1) Polysaccharide monolithic structure

In general, monolithic structure means a single block of porous body structure continuously having three-dimensional network structure skeletons and voids, respectively.

A monolithic structure according to the invention is a thick stereoscopic porous body including a polysaccharide as a main component, and having continuous pores.

Specifically, the monolithic structure according to the invention has continuous pores having an average pore diameter of approximately 0.01 to approximately 20.0 micrometers. The average pore diameter can be determined from an image photographed using a scanning electron microscope (SEM).

Here, the continuous pores mean continuous voids, and not individually independent cavities. The pores being continuous can be imagined from observation of an identical or similar shape in a SEM photograph of a plurality of monolithic structure samples.

With regard to the shape of pores, the pores on a structure surface and/or inside the structure are preferably circular or elliptical or approximate thereto, but are not particularly limited.

The monolithic structure according to the invention has such continuous pores, thereby being excellent in liquid permeability. Moreover, an arbitrary amount of a functional group or a ligand can be introduced thereinto, and reaction efficiency in the functional group or the ligand is satisfactory. Therefore, the monolithic structure is suitable for a column material used for a purification method or the like as described later.

The monolithic structure according to the invention has a thickness of approximately 100 micrometers or more, and preferably, approximately 150 micrometers or more, and has a stereoscopic form different from a membrane. In the invention, a shape of the porous body is not limited, but length in a shortest axis direction is referred to as thickness, for convenience, among three directions of depth, width and height of the porous body. Moreover, the monolithic structure according to the invention can be formed into an arbitrary shape such as a columnar shape and a cylindrical shape.

Specific examples of the polysaccharide mainly constituting the monolithic structure according to the invention include cellulose and glucomannan. Moreover, the polysaccharide includes a polysaccharide derivative, and may also be an esterified polysaccharide, for example. Specific examples of the esterified polysaccharide include cellulose acetate, cellulose acetate propionate, cellulose acetate butyrate and glucomannan acetate.

A molecular weight of the polysaccharide described above is not particularly limited, but in a case of cellulose acetate, for example, an average molecular weight (Mw) is preferably approximately 1,000 to approximately 200,000, and further preferably, approximately 5,000 to approximately 100,000 from a viewpoint of handling properties in the manufacturing method according to the invention as described later, strength of a formed monolithic structure or a diameter of holes of the monolithic structure.

Adoption of the polysaccharide, particularly, cellulose as a constitutional material of the monolithic structure allows formation of a column material that can further withstand alkali washing in comparison with silica being a conventional column material. Moreover, the polysaccharide has small nonspecific adsorption, and therefore is suitable as a chromatography base material. Moreover, the polysaccharide has usability as a chromatography base material also in view of the polysaccharide having many hydroxyl groups and ease of introduction of a functional group or a ligand thereinto. The functional group or the ligand can also be introduced into a hydroxyl group in silica. However, an introduction procedure is complicated, and therefore the introduction is not so practical.

In the polysaccharide monolithic structure according to the invention, the polysaccharide may be modified with various functional groups, and such modification is ordinarily applied to at least one hydroxyl group of the polysaccharide.

For example, a hydroxyl group of a saccharide may be esterified, thereby allowing suppression of the nonspecific adsorption. As ester, an acetyl group is preferred, for example. Alternatively, an acetylated hydroxyl group of the saccharide may be further deacetylated.

Moreover, saccharide chains may be cross-linked therebetween, thereby allowing improvement in strength of the monolithic structure.

Moreover, in the polysaccharide monolithic structure according to the invention, various arbitrary ligands may be introduced into the polysaccharide, and such introduction is ordinarily applied to at least one hydroxyl group of the polysaccharide. The ligand is introduced thereinto, thereby allowing provision of adsorption performance to an objective substance such as a biopolymer for the polysaccharide monolithic structure to allow suitable utilization for purification or detection of the objective substance as described later.

The ligand is not particularly limited, and specific examples include an ion exchange group such as 2-diethylamino ethyl chloride hydrochloride (DEAE), CM, a sulfone group, quaternary ammonium and sulfate, a ligand for protein adsorption, such as protein A and an antibody, and a functional polymer such as e-polylysine and γ-polylysine, and the ligand can be arbitrarily selected according to a purpose of using the monolithic structure.

### (2) Manufacturing method for polysaccharide monolithic structure

The polysaccharide monolithic structure according to the invention can be manufactured by a method including a first step for dissolving a polysaccharide into a mixed solvent including a solvent into which the polysaccharide is soluble and a solvent into which the polysaccharide is insoluble, at a temperature lower than a boiling point of the mixed solvent, to give a polysaccharide solution, and a second step for cooling the polysaccharide solution to give the polysaccharide monolithic structure.

The polysaccharide used as a material includes a polysaccharide derivative, and specific examples of a preferred material include esterified cellulose and esterified glucomannan. Specific examples preferably include cellulose acetate, cellulose acetate propionate, cellulose acetate butyrate and glucomannan acetate, and cellulose acetate is particularly preferred among the derivatives.

A molecular weight of the polysaccharides used as the material is not particularly limited, but in a case of cellulose acetate, for example, an average molecular weight (Mw) is approximately 1,000 to approximately 200, 000, and further preferably, approximately 5, 000 to approximately 100,000 from a viewpoint of handling properties in the manufacturing step, and strength of a formed monolithic structure or a pore diameter of holes of the monolithic structure. Cellulose acetate having an average molecular weight of 40, 000 is available from Wako Pure Chemical Industries, Ltd., and can be preferably used.

The first step in the manufacturing method according to the present invention includes a step for dissolving a polysaccharide in a mixed solvent of a solvent (hereinafter, referred to as a good solvent) into which the polysaccharide is soluble, and a solvent (hereinafter, referred to as a poor solvent) into which the polysaccharide is insoluble, at a temperature lower than a boiling point of the mixed solvent, to give a polysaccharide solution.

Thus, the mixed solvent of the good solvent and the poor solvent is adopted as a solvent for dissolving the polysaccharide thereinto, thereby allowing easy manufacture of a desired polysaccharide monolithic structure.

Here, the good solvent means a solvent into which a solute dissolves to give a transparent solution containing no solid matter. Further, the good solvent means a solvent to give a solution having a concentration of approximately 1% by weight or more, and further, approximately 10% by weight or more, at room temperature herein. The poor solvent means a solvent having no capability of dissolving a polysaccharide in approximately 0.0001% by weight or more by itself at room temperature.

When the polysaccharide includes an esterified polysaccharide, specific examples of the good solvent include ketones such as acetone and methyl ethyl ketone, esters or cyclic esters, such as methyl acetate, ethyl acetate and γ-butyrolactone, a nitrogen-containing compound such as dimethylformamide and N-methyl pyrrolidone, glycols such as methyl glycol and methyl glycol acetate, ethers such as tetrahydrofuran and dioxane, a halogenated hydrocarbon such as chloroform and dichloromethane, and also dimethyl sulfoxide, or a combination thereof.

When the polysaccharide includes the esterified polysaccharide, specific examples of the poor solvent include alcohols such as methanol and ethanol, and water, or a combination thereof.

With regard to a mixing ratio of the good solvent and the poor solvent, a ratio of the good solvent is further preferably approximately 5 to approximately 60 v/v% and a ratio of the poor solvent is further preferably approximately 95 to approximately 40 v/v%. If a ratio of the good solvent exceeds approximately 60 v/v%, precipitation by phase separation may be occasionally hard to take place even if the polysaccharide solution is cooled. Moreover, if the ratio of the good solvent is smaller than approximately 5 v/v%, the mixed solvent may occasionally allow no uniform dissolution of the polysaccharide.

Moreover, a ratio of the polysaccharide in the mixed solvent is preferably approximately 0.1 to approximately 30% by weight, and further preferably approximately 1 to approximately 20% by weight. When the ratio exceeds 30% by weight, a uniform solution is hard to be obtained and the porous body having well-aligned continuous pores is hard to be obtained. When the ratio is less than approximately 0.1% by weight, precipitation by phase separation may be occasionally hard to take place even if the polysaccharide solution is cooled, and even if the precipitation takes place, the monolithic structure has insufficient strength to allow no maintenance of a shape.

Moreover, a larger ratio of the polysaccharides in the mixed solvent tends to further decrease the pore diameter of the continuous pores of the porous body obtained. Therefore, when the porous body is applied to the chromatography described later, the ratio of the polysaccharide in the mixed solvent is adjusted to be preferably approximately 5 to approximately 30% by weight, and further preferably, approximately 5 to approximately 20% from a viewpoint of forming a pore diameter to give satisfactory liquid permeability.

The first step in the manufacturing method according to the invention includes the step for dissolving the polysaccharide into the mixed solvent of the good solvent and the poor solvent at a temperature lower than the boiling point of the mixed solvent to give the polysaccharide solution, but a third component soluble in the mixed solvent is added herein, thereby allowing an increase in the pore diameter of the continuous pores of the porous body obtained.

As the third component, any component that is dissolved into any of the good solvent and the poor solvent of the polysaccharide may be satisfactorily used, and a component dissolved into the mixed solvent may be satisfactorily used.

Specific examples of the third component include a component such as a water-soluble polymer including polyethylene glycol (PEG) and polyvinyl alcohol (PVA), saccharides including glucose, sucrose and starch, and salts including calcium chloride and calcium carbonate. Specifically, polyethylene glycol is preferred.

A molecular weight of the polyethylene glycol is not particularly limited, but an average molecular weight (Mw) is preferably approximately 100 to approximately 10,000, and further preferably, approximately 1, 000 to approximately 5, 000 from a viewpoint of handling properties in the manufacturing step, and strength of a formed monolithic structure or a pore diameter of holes of the monolithic structure. Polyethylene glycol having an average molecular weight of 3, 000 is available from Wako Pure Chemical Industries, Ltd. , and can be preferably used.

The second step in the manufacturing method according to the invention includes a step for cooling the polysaccharide solution obtained in the first step to give the polysaccharide monolithic structure.

In the step, phase separation takes place to give a precipitated monolithic structure. The monolithic structure obtained herein includes the mixed solvent inside the pores thereof.

In the second step, the polysaccharide solution is cooled to a temperature lower by approximately 5 to approximately 200°C, preferably, to a temperature lower by approximately 5°C to approximately 150°C, and further preferably, to a temperature lower by approximately 5 to approximately 200°C, than a temperature before cooling (temperature during the dissolution in the first step). A temperature dropping rate on the above occasion is preferably approximately 0.1 to approximately 200°C per minute, and further preferably, approximately 0.1 to approximately 100°C per minute.

The monolithic structure according to the invention is obtained through the first step and the second step. However, treatment described below may be further applied, when necessary, after the second step.

As described above, the monolithic structure after the second step includes the mixed solvent used in the first step inside thereof. Therefore, the mixed solvent may be replaced by an arbitrary solvent. A method of replacement is not particularly limited. For example, the monolithic structure after the second step is immersed into an arbitrary solvent in another container, thereby allowing replacement by mutual diffusion. Here, a solvent for replacement needs to be miscible with the solvent used in the first step in order to keep homogeneity of the monolithic structure obtained in the second step. Specific examples of a preferred solvent include alcohols and water.

Moreover, treatment may be applied for removing the solvent contained inside the monolithic structure. An ordinary method such as heating and pressure reduction can be applied as the method for removal.

Moreover, when the esterified polysaccharide is used as a material in the first step, saponification (deesterification) may be applied as a third step after the second step. The saponification treatment can be arbitrarily applied in accordance with an ordinary method such as alkali treatment.

Furthermore, a fourth step for introducing an arbitrary functional group or ligand into a free hydroxyl group may be applied to the saponified (deesterified) monolithic structure after the third step. Thus, the monolithic structure provided with adsorption performance to an objective substance such as a biopolymer can be obtained.

The ligand is not particularly limited, and specific examples include an ion exchange group such as 2-diethylaminoethyl chloride hydrochloride (DEAE), carboxymethyl (CM), a sulfone group, quaternary ammonium and sulfate, a hydrophobic group such as a phenyl group and a butyl group, a ligand that can be used for separation of a so-called mixed mode having both an ion exchange group and a hydrophobic group, a ligand for protein adsorption, such as protein A and an antibody, and a functional polymer such as a polycation including polylysine, a polyanion including heparin and polyglutamic acid. The ligand can be arbitrarily selected according to a purpose of using the monolithic structure, and an introducing method thereof can be arbitrarily applied in accordance with an ordinary method.

Moreover, as the fourth step, treatment for cross-linking a polysaccharide may be applied in addition to or in place of introduction of a functional group or ligand. Cross-linking treatment can be arbitrarily applied using a reactive bifunctional reagent as a cross-linking agent in accordance with an ordinary method. Specific examples of the reactive bifunctional reagent preferably include epichlorohydrin, epibromohydrin, diisocyanate, dimethylurea, dimethylethyleneurea, dimethylchlorosilane, bis(2-hydroxyethylsulfone), butanediol diglycidyl ether, ethylene glycol diglycidyl ether, glycerol diglycidyl ether, polyethylene glycol diglycidyl ether, divinylsulfone, alkylene dihalogen and hydroxy alkylene dihalogen, and among the compounds, epichlorohydrin can be particularly preferably used.

### (3) Purification method using monolithic structure

The monolithic structure according to the invention can be used for chromatography in a form of a column or the like.

The monolithic structure according to the invention has continuous pores, and therefore pressure in the column is hard to increase during liquid permeation. Therefore, the monolithic structure allows maintenance or increase of a flow rate, and accordingly is suitable for execution of chromatography.

Moreover, as described above, a desired functional group or ligand can be introduced into the monolithic structure according to the invention, and therefore the structure allows provision of adsorption performance to a biopolymer. Various objective substances can be purified using the performance. For example, a monolithic structure into which the ion exchange group is introduced can be used for purifying expression protein, a monolithic structure in which protein A is immobilized as a ligand can be applied to IgG purification, or a monolithic structure in which an antibody is immobilized can be applied to detection or purification of an antigen corresponding to the antibody.

It will be apparent to those skilled in the art that various modifications and variations can be made in the invention and specific examples provided herein without departing from the spirit or scope of the invention. Thus, it is intended that the invention covers the modifications and variations of this invention, that come within the scope of any claims and their equivalents.

The following examples are for illustrative purposes only and are not intended, nor should they be interpreted to, limit the scope of the invention.

### Examples

In the following, the invention is explained in more detail by way of Examples, but the invention is not limited thereto.

### Manufacture Example 1

In a transparent test tube, 200 mg of cellulose acetate (5% by weight) (Mw: 40,000, made by Wako Pure Chemical Industries, Ltd.) was added to a mixed solvent of 0.8 mL (18 v/v%) of 1,4-dioxane, 2.8 mL (65 v/v%) of ethanol and 0.7 mL (17 v/v%) of water, and the resulting mixture was heated and dissolved thereinto in a water bath at 60°C to give a polysaccharide solution. The resulting polysaccharide solution was cooled to room temperature (approximately 26°C) to give a monolithic structure including a solvent. The resulting monolithic structure including the solvent was charged into an excess amount of water in another container to replace the mixed solvent included in the monolithic structure by water. The monolithic structure obtained had a columnar shape having a diameter of 13 mm and a length of approximately 25 mm. Subsequently, a monolithic structure including water was removed from water, frozen at -78°C, and then dried under reduced pressure at room temperature to remove water inside the structure.

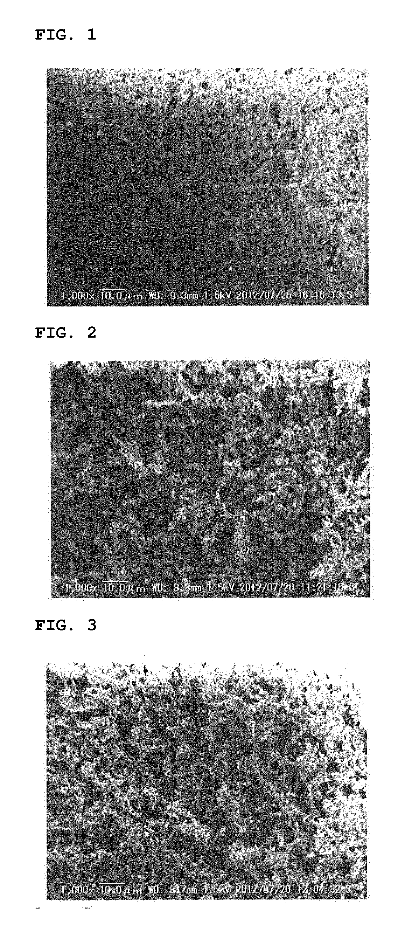

FIG. 1 shows a micrograph obtained by observing a cross section of the resulting monolithic structure by a scanning electron microscope (SEM) (VE-8800, made by KEYENCE CORPORATION).

### Manufacture Example 2

Then, 250 mg of cellulose acetate was added to a mixed solvent of 1.5 mL (30 v/v%) of 1,4-dioxane, 3.0 mL (60 v/v%) of ethanol and 0.5 mL (10 v/v%) of water, and the resulting mixture was heated and dissolved thereinto in a water bath at 60°C to give a polysaccharide solution. The resulting polysaccharide solution was cooled to room temperature (approximately 5°C) to give a monolithic structure including a solvent. The resulting monolithic structure including the solvent was charged into an excess amount of water in another container to replace the mixed solvent included in the monolithic structure by water. The monolithic structure obtained had a columnar shape having a diameter of 13 mm, and a length of approximately 30 mm.

### Manufacture Example 3

A columnar monolithic structure was obtained in a manner similar to Manufacture Example 1 except that a composition of the mixed solvent was changed to 1.2 mL (30 v/v%) of 1,4-dioxane, 1.4 mL (35 v/v%) of ethanol and 1.4 mL (35 v/v%) of water.

### Manufacture Example 4

A columnar monolithic structure was obtained in a manner similar to Manufacture Example 1 except that a process of cooling a polysaccharide solution was applied in a water bath at 35°C. FIG. 2 shows a SEM photograph of a cross section of the monolithic structure.

### Manufacture Example 5

A columnar monolithic structure was obtained in a manner similar to Manufacture Example 1 except that a process of cooling a polysaccharide solution was applied in a water bath at 30°C. FIG. 3 shows a SEM photograph of a cross section of the monolithic structure.

### Manufacture example 6

A columnar monolithic structure was obtained in a manner similar to Manufacture Example 1 except that a process of cooling a polysaccharide solution was applied in a water bath at 0°C.

### Manufacture Example 7

In a transparent test tube, 300 mg of cellulose acetate was added to a mixed solvent of 1.0 mL (33 v/v%) of chloroform and 2.0 mL (67 v/v%) of methanol, and the resulting mixture was heated and dissolved thereinto in a water bath at 50°C to give a polysaccharide solution. The resulting polysaccharide solution was treated in a manner similar to Manufacture Example 1 to give a columnar monolithic structure.

### Manufacture Example 8

A columnar monolithic structure having a diameter of 13 mm and a length of approximately 25 mm was obtained in a manner similar to Manufacture Example 1 except that a composition of a mixed solvent was changed to 0.8 mL (20 v/v%) of N-methylpyrrolidone, 2.0 mL (50 v/v%) of ethanol and 1.2 mL (30 v/v%) of water. The monolithic structure including water was removed from water, frozen at -78°C, and then dried under reduced pressure at room temperature to remove water inside the structure.

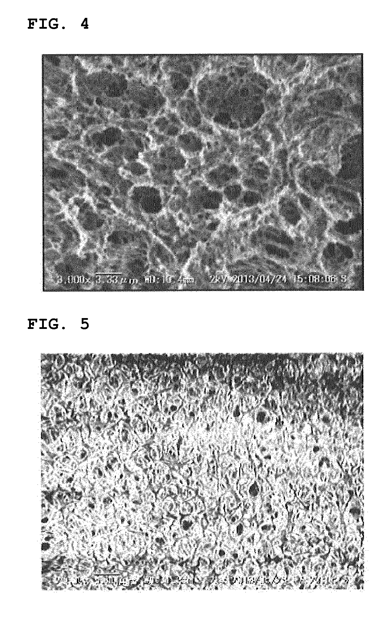

FIG. 4 shows a photograph obtained by observing a cross section of the resulting monolithic structure by a scanning electron microscope.

### Manufacture Example 9

A columnar monolithic structure was obtained in a manner similar to Manufacture Example 1 except that a composition of a mixed solvent was changed to 2.0 mL (50 v/v%) of N-methylpyrrolidone, 0.8 mL (20 v/v%) of ethanol and 1.2 mL (30 v/v%) of water.

### Manufacture example 10

A columnar monolithic structure was obtained in a manner similar to Manufacture Example 1 except that a composition of a mixed solvent was changed to 1.8 mL (34 v/v%) of γ-butyrolactone, 2.8 mL (53 v/v%) of ethanol and 0.7 mL (13 v/v%) of water.

### Manufacture Example 11

A columnar monolithic structure was obtained in a manner similar to Manufacture Example 1 except that a composition of a mixed solvent was changed to 1.9 mL (35 v/v%) of dimethyl sulfoxide, 2.8 mL of ethanol (52 v/v%) and 0.7 mL (13 v/v%) of water.

### Manufacture Example 12

A monolithic structure was obtained in a manner similar to Manufacture Example 7 except that 1.0 g of acetylated glucomannan was weighed in a 50 mL sample tube and dissolved into 10 mL (45 v/v%) of chloroform and 10 mL (55 v/v%) of ethanol. The monolithic structure obtained had a columnar shape having a diameter of 2.4 cm and a length of 1.5 cm.

### Manufacture example 13

In a 100 mL recovery flask, 2.5 g of cellulose acetate was added to a mixed solvent of 15 mL (30 v/v%) of 1,4-dioxane, 30 mL (73 v/v%) of ethanol and 5.0 ml (10 v/v%) of water, and the resulting mixture was heated and dissolved thereinto in a water bath at 60°C to give a polysaccharide solution. Then, the resulting polysaccharide solution was dispensed by 4 mL to a test tube (1.5 cm in diameter, 10 cm in height). The polysaccharide solution was cooled to 10°C to give a monolithic structure including a solvent. The resulting monolithic structure including the solvent was charged into an excess amount of water in another container to replace the mixed solvent included the monolithic structure by water. The monolithic structure obtained had a columnar shape having a diameter of 1.3 cm and a length of 2.2 cm.

### Manufacture Example 14

In a transparent test tube, 500 mg (10% by weight) of cellulose acetate was added to a mixed solvent of 1. 5 mL (30 v/v%) of 1, 4-dioxane, 3.0 mL (60 v/v%) of ethanol and 0. 5 mL (10 v/v%) of an aqueous solution of 20 wt% polyethylene glycol (Mw: 3,000, made by Wako Pure Chemical Industries, Ltd.), and the resulting mixture was heated and dissolved thereinto in a water bath at 60°C to give a polysaccharide solution. The resulting polysaccharide solution was cooled to 20°C to give a monolithic structure including a solvent. The resulting monolithic structure including the solvent was charged into an excess amount of water in another container to replace the mixed solvent included in the monolithic structure by water, and remove polyethylene glycol. The monolithic structure obtained had a columnar shape having a diameter of 13 mm and a length of 25 mm.

### Manufacture Example 15

In a 100 mL recovery flask, 2.5 g of cellulose acetate was added to a mixed solvent of 15 mL (30 v/v%) of 1,4-dioxano, 30 mL (73 v/v%) of ethanol and 5.0 ml (10 v/v%) of water, and the resulting mixture was heated and dissolved thereinto in a water bath at 60°C to give a polysaccharide solution. Then, the resulting polysaccharide solution was dispensed by 4 mL into a test tube (1.5 cm in diameter, 10 cm in height). The polysaccharide solution was cooled to 10°C to give a monolithic structure including a solvent. The resulting monolithic structure including the solvent was charged into an excess amount of ethanol in another container to replace the mixed solvent included in the monolithic structure by ethanol.

Into 3 mL of ethanol, 1 mL of 10 M sodium hydroxide solution was added and the resulting mixture was sufficiently mixed in a test tube. Thereto, one of the monolithic structures replaced by ethanol (approximately 200 mg in dry weight) as previously obtained was put and shaken at 30°C for 5 hours (100 rpm) to perform deacetylation. Then, the resulting monolithic structure was immersed into water and the solvent was replaced.

Then, 320 µL of epichlorohydrin was added to 1.5 mL of water, and the resulting mixture was sufficiently mixed in a test tube. The monolithic structure after deacetylation as previously obtained was added thereto, and the resulting mixture was shaken at 30°C for 3 hours. Subsequently, 0.16 mL of epichlorohydrin, 1.5 mL of water and 0.42 mL of 10 M NaOH were put in another test tube, and the resulting mixture was sufficiently mixed. Here, the monolith immersed into the epichlorohydrin aqueous solution was added, and the resulting mixture was stirred at 50°C for 15 hours to perform a cross-linking reaction. After reaction completion, the monolithic structure was charged into water, and washed with water until the aqueous solution became neutral to replace the solvent inside the structure by water. The monolithic structure obtained had a columnar shape having a diameter of 1.2 cm and a length of 1.9 cm.

### Manufacture Example 16

In a test tube, 0.36 g of 2-diathylamina ethyl chloride hydrochloride (Wako Pure Chemical Industries, Ltd., hereinafter referred to as DEAE) was dissolved into 1.5 mL of ion-exchanged water, the monolith obtained in Manufacture Example 15 was immersed thereinto, and the resulting mixture was shaken at 30°C for 3 hours (100 rpm). Then, 1.0 mL of water and 0.52 mL of 10 M NaOH were put in another test tube and sufficiently mixed. Thereinto, the monolith that immersed into the DEAE aqueous solution was immersed, the resulting mixture was shaken at 50°C for 15 hours to perform a DEAE introduction reaction. After reaction completion, the monolithic structure was charged into water, and washed with water until rinsed water became neutral to replace the solvent inside the structure by water. The monolithic structure obtained had a columnar shape having a diameter of 1.2 cm and a length of 2.0 cm.

With regard to the monolithic structure obtained in Manufacture Example 16, neutralization titration was performed to measure an amount of DEAE introduced (ion exchange capacity) according to procedures described below.

A monolith was put in a centrifugal tube and immersed into a 0.5 M NaOH aqueous solution, and the resulting mixture was shaken for 18 hours. Next, the monolith was washed with MilliQ water until rinsed water became neutral. Excessive moisture deposited on the monolith was wiped off, and weight was measured, and the moisture content was determined. Water and 0.5 M HCl were added to the monolith to be a 0.1 M HCl solution in a final concentration including the moisture content, and the monolith was immersed thereinto for 16 hours. Then, 2 mL of immersion liquid was measured using a transfer pipette and titrated with 0.1 M NaOH. The result showed that the amount of DEAE introduction was 1.0 meg/g-dry.

### Manufacture Example 17

In a 100 mL recovery flask, 5.0 g of cellulose acetate was added to a mixed solvent of 15 mL (30 v/v%) of 1,4-dioxane, 30 mL (60 v/v%) of ethanol and 5.0 mL (10 v/v%) of 20 wt% polyethylene glycol aqueous solution, and the resulting mixture was heated and dissolved thereinto in a water bath at 60°C to give a polysaccharide solution. Then, the resulting polysaccharide solution was dispensed by 5.0 mL into a test tube (1.5 cm in diameter, 10 cm in height). The polysaccharide solution was cooled to 20°C to give a monolithic structure including a solvent. The resulting monolithic structure including the solvent was charged into an excess amount of water in another container to replace the mixed solvent included in the monolithic structure by water. One of the monolithic structures including water was removed from water, frozen at -78°C, and then dried under reduced pressure at room temperature to remove water inside the structure. FIG. 5 shows a photograph obtained by observing a cross section of the resulting monolithic structure by a scanning electron microscope.

To 2.5 mL of water, 2.5 mL of 10 M sodium hydroxide solution was added and the resulting mixture was sufficiently mixed in a test tube. One of the monolithic structures replaced by water (approximately 400 mg in dry weight) as previously obtained was put therein and shaken at 30°C for 5 hours (100 rpm) to perform deacetylation. Then, the resulting monolithic structure was immersed into water, and washed with water until the aqueous solution became neutral.

Then, 350 µL of epichlorohydrin was added to 5.0 mL of water, and the resulting solution was sufficiently mixed in a test tube. The monolithic structure after deacetylation as previously obtained was added thereto, and the resulting mixture was shaken at 30°C for 2 hours. Subsequently, 2.75 mL of water and 0.25 mL of 10 M NaOH were put in another test tube, and the resulting mixture was sufficiently mixed. The monolith immersed into the epichlorohydrin aqueous solution was added thereto, and the resulting mixture was stirred at 50°C for 2 hours to perform a cross-linking reaction. After reaction completion, immersion in an epichlorohydrin aqueous solution (350 microliters of epichlorohydrin / 5 mL of water), and a cross-linking reaction in a 10 M NaOH aqueous solution (0.25 mL of 10 M NaOH / 2.75 mL of water) was again performed in a manner as previously described. Then, the monolithic structure was put in water, and washed with water until the aqueous solution became neutral to replace the solvent inside the structure by water. The monolithic structure obtained had a columnar shape having a diameter of 1.2 cm and a length of 2.1 cm.

### Manufacture Example 18

In a test tube, 1.4 g of DEAE was dissolved into 2.0 mL of ion-exchanged water, the cross-linked monolith obtained in Manufacture Example 17 was immersed thereinto, and the resulting mixture was shaken at 30°C for 3 hours (100 rpm). Then, 1.3 mL of water and 1.7 mL of 10 M NaOH were put in another test tube, and the resulting mixture was sufficiently mixed. The monolith immersed into the DEAE aqueous solution was immersed thereinto, and the resulting mixture was shaken at 50°C for 15 hours to perform a DEAE introduction reaction. After reaction completion, the monolithic structure was charged into water, and washed with water until rinsed water became neutral to replace the solvent within the structure by water. The monolithic structure obtained had a columnar shape having a diameter of 1.2 cm and a length of 2.2 cm.

### Reference Manufacture Example

In a transparent test tube, 0.25, 0.5 or 1.0 g (5, 10 or 20% by weight) of cellulose acetate, and 4.75, 4.5 or 4 g of 2-ethyl-1,3-hexandiol being a solvent used for a manufacturing method for a cellulose porous membrane as described in Patent literature No. 4 were added, respectively, and heated and dissolved thereinto by a block heater at 150°C to give a polysaccharide solution. When the resulting polysaccharide solution was cooled to 20°C, fluidity was found in a case of 5% by weight, and no formed body as a gel was obtained. Moreover, in a case of 10% by weight, when the solution was cooled, deposition took place and inhomogeneous precipitation was observed. Moreover, in a case of 20% by weight, a three-dimensional structure having a diameter of 1.5 cm and a length of 3.2 cm was obtained, but liquid permeability performance was very poor and a pore diameter was also small.

### Test Exantple 1 Evaluation of continuous pores

In order to investigate continuity of pores in the cellulose acetate monolithic structure in Manufacture Example 1, a column was assembled according to procedures described below.

A rod-shaped cellulose acetate monolithic structure having a diameter of 0.9 cm was cut into a desired length with a cutter. Both ends of the monolith were interposed between a 2.5 cm TUBING PTFE (I. D.: 7, O. D.: 9 mm) (purchased from AS ONE Corporation) and a polypropylene frit (SUPELCO, made by Sigma-Aldrich Japan K. K.) having a diameter of 9.3 mm and a thickness of 2.4 mm, and the resulting set was further put in a Teflon (registered trademark) heat-shrinkable tube FEP-090 (purchased from AS ONE Corporation) having a length with which a full length of the set was covered, and the whole was heated in a dryer to cause shrinkage. Profile unions (30-10RU6-C, made by SANPLATEC CO., LTD.) were connected to both ends of the resulting column. A tube having an outer diameter of 6 mm was connected thereto to measure a flow rate. Furthermore, the resulting column was used for measurement by a chromatography system by further connecting a 1/16 inch (1.59 mm) tube to a profile union (U-20C, Tokyo Rikakikai Co., Ltd.).

An aqueous solution of NaCl, bovine serum albumin (BSA) or iron colloid (average particle diameter: 78 nm) was allowed to permeate through the column, respectively, and a recovery rate of an eluted substance was investigated.

As the chromatography system, BioLogic LP (Bio-Rad Laboratories, Inc.) was used, and NaCl was detected by electrical conductivity, and BSA and iron colloid were detected by absorption at 280 nm, respectively. The flow rate was adjusted to 1.2 mL/min. The monolithic structure used had a diameter of 0.9 cm and a length of 2.5 cm.

In a case of NaCl, the column was equilibrated with pure water, and then 10 mL of 1 M NaCl aqueous solution was allowed to permeate through the column. For comparison, a tube was directly connected without connecting the column, and a 1 M NaCl aqueous solution was allowed to permeate therethrough in a similar manner. Elution time and electrical conductivity were recorded by a chart recorder, respectively. Each peak area was determined using digitizer software (UN-SCAN-IT).

In a case of BSA and iron colloid, a column was equilibrated with PBS (-) (Dulbecco's PBS (-) made by NISSUI PHARMACEUTICAL CO., LTD. was prepared according to an operation manual), and then 2 mL of PBS (-) solution of 1% BSA or 20 µg/mL iron colloid was allowed to permeate through the column, respectively. Each of elution time and absorbance at 280 nm was recorded by a chart recorder. Each peak area was determined using digitizer software (UN-SCAN-IT).

In all cases, measurement was repeated twice, and a recovery rate was calculated from an average value.

Table 1 shows the results. In any of samples of NaCl, BSA and iron colloid, an amount of recovery became substantially 100%, and the pores of the monolithic structure in Manufacture Example 1 were confirmed to be continuous.

**Table 1 Substance permeability of monolith column**

| Sample | Peak area | | Recovery rate (%) |
|---|---|---|---|
| | Tube only | Liquid permeation through a column | |
| 1 M NaCl | 500.0 | 510.3 | 102.1 |
| 1% BSA | 109.3 | 105.3 | 96.3 |
| 20 µg/mL iron colloid | 87.5 | 88.2 | 100.8 |

### Test Example 2 Evaluation of relationship between flow rate and pressure during liquid permeation

Pure water was allowed to permeate through the cellulose acetate monoliths in Manufacture Example 13 and Manufacture Example 14, and pressure at a column inlet on the occasion was measured. First, a column was assembled according to procedures described below.

A rod-shaped cellulose acetate monolithic structure having a diameter of 1.2 cm was cut into a desired length with a cutter. Both ends of the monolith were interposed between a 5.0 cm TUBING PTFE (I. D. : 7, O. D. : 9 mm) and a polypropylene frit having a diameter of 9.3 mm and a thickness of 2.4 mm, and the resulting set was further put in SUMITUBE C (SUMI-C-14, SUMITOMO ELECTRIC FINE POLYMER, INC.) having a length with which a full length of the set was covered, and the whole was heated in a water bath at 95°C to cause shrinkage. The resulting set was further put in a Teflon (registered trademark) heat-shrinkable tube FEP-120 (purchased from AS ONE Corporation) having a length with which a full length of the set was covered, and the whole was heated in a dryer to cause shrinkage. Profile unions (30-10RU6-C, made by SANPLATEC CO., LTD.) were connected to both ends of the resulting column. A tube having an outer diameter of 6 mm was connected thereto to measure a flow rate. Moreover, the resulting column was used for measurement by a chromatography system by further connecting a 1/16 inch (1.59 mm) tube to a profile union (U-20C, Tokyo Rikakikai Co., Ltd.).

As a measuring instrument, LC8A (Shimadzu) was used as a pump, GP-M025 (KEYENCE) was used as a pressure gauge and FD-SS02A (KEYENCE) was used as a flowmeter. The monolithic structures used had a diameter of 0.9 cm and a length of 2.5 cm. As a liquid to be permeated through the column, pure water at 31 to 32°C was used.

As Comparative Example, Cellufine MAX S-r Mini-Column (diameter: 0.9 cm, length: 1.8 cm, made by JNC CORPORATION) being a column packed with cellulose particles was used, and an evaluation was made in a similar manner.

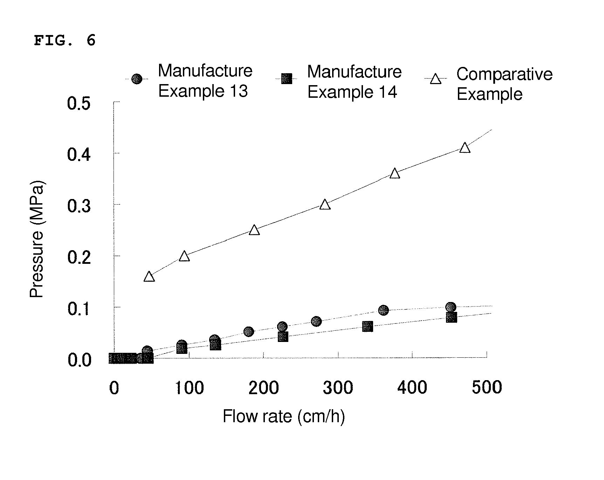

FIG. 6 shows the results. The monolithic structures in Manufacture Example 13 and Manufacture Example 14 are found to allow liquid permeation at a by far lower pressure and a higher flow rate in comparison with the column packed with particles.

Although the invention has been described and illustrated with a certain degree of particularity, it is understood that the disclosure has been made only by way of example, and that numerous changes in the conditions and order of steps can be resorted to by those skilled in the art without departing from the spirit and scope of the invention.

### Industrial Applicability

The invention can provide a monolithic structure having a pore diameter suitable for biopolymer separation from an inexpensive polysaccharide or polysaccharide derivative. Moreover, the invention can arbitrarily provide the monolithic structure with adsorption performance to a biopolymer to contribute to improvement in efficiency in a purification step in manufacturing a medicine or the like, and therefore is industrially very useful.

## Claims

1. A polysaccharide-containing monolithic structure, wherein the polysaccharide monolithic structure is a porous body having continuous pores having an average pore diameter of 0.01 to 20 micrometers, and
a thickness of 100 micrometers or more.

2. The polysaccharide monolithic structure according to claim 1, wherein the polysaccharide is cellulose.

3. The polysaccharide monolithic structure according to claim 1 or 2, wherein at least one hydroxyl group of the polysaccharide is esterified.

4. The polysaccharide monolithic structure according to any one of claims 1 to 3, comprising cross-linked structure.

5. The polysaccharide monolithic structure according to any one of claims 1 to 4, wherein a ligand is introduced into at least one hydroxyl group of the polysaccharide.

6. A manufacturing method for a polysaccharide monolithic structure, comprising:
a first step for dissolving a polysaccharide into a mixed solvent including a solvent into which the polysaccharide is soluble and a solvent into which the polysaccharide is insoluble, at a temperature lower than a boiling point of the mixed solvent, to give a polysaccharide solution; and
a second step for cooling the polysaccharide solution to give the polysaccharide monolithic structure.

7. The manufacturing method according to claim 6, wherein a third component is added to the mixed solvent.

8. The manufacturing method according to claim 7, wherein a concentration of the third component in the mixed solvent is 0.1 to 5% by weight.

9. The manufacturing method according to claim 7 or 8, wherein the third component is polyethylene glycol.

10. The manufacturing method according to any one of claims 6 to 9, wherein a volume ratio of the solvent into which the polysaccharide is soluble and the solvent into which the polysaccharide is insoluble in the mixed solvent is 5 : 95 to 60 : 40.

11. The manufacturing method according to any one of claims 6 to 10, wherein a concentration of the polysaccharide in the mixed solvent is 0.1 to 30% by weight.

12. The manufacturing method according to any one of claims 6 to 11, wherein, in the second step, the polysaccharide solution is cooled to a temperature lower by 5 to 200°C than a temperature before cooling.

13. The manufacturing method according to any one of claims 6 to 12, wherein the polysaccharide is esterified cellulose.

14. A manufacturing method for a saponified polysaccharide monolithic structure, comprising a third step, after a polysaccharide monolithic structure is manufactured by the manufacturing method according to any one of claims 6 to 13, to be applied for saponifying the polysaccharide monolithic structure after the second step.

15. A manufacturing method for a cross-linked and/or ligand-introduced polysaccharide monolithic structure, comprising a fourth step, after a saponified polysaccharide monolithic structure is manufactured by the manufacturing method according to claim 14, to be applied for applying cross-linking and/or ligand-introducing treatment after the third step.

16. A purification method for an objective substance, using the polysaccharide monolithic structure according to any one of claims 1 to 5.
